(19) Europäisches Patentamt
European Patent Office
Office européen des brevets

(11) EP 4 127 723 B1

(12) **EUROPEAN PATENT SPECIFICATION**

(45) Date of publication and mention of the grant of the patent:
**11.06.2025 Bulletin 2025/24**

(21) Application number: **21713692.8**

(22) Date of filing: **25.03.2021**

(51) International Patent Classification (IPC):
***G01N 33/574*** *(2006.01)*

(52) Cooperative Patent Classification (CPC):
**G01N 33/57438;** G01N 2400/00; G01N 2440/38; G01N 2800/245; G01N 2800/52

(86) International application number:
**PCT/EP2021/057788**

(87) International publication number:
**WO 2021/191372 (30.09.2021 Gazette 2021/39)**

(54) **PROGNOSTIC MARKERS OF DISEASE RECURRENCE IN LIVER TRANSPLANT RECIPIENTS WITH HEPATOCELLULAR CARCINOMA**

PROGNOSTISCHE KRANKHEITSMARKER IN LEBERTRANSPLANTATIONSEMPFÄNGERN MIT HEPATOZELLULÄREM KARZINOM

MARQUEURS DE PRONOSTIC DE LA RÉCURRENCE DE LA MALADIE CHEZ LES RECEVEURS DE GREFFE DE FOIE ATTEINTS D’UN CARCINOME HÉPATOCELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2020 EP 20166316**

(43) Date of publication of application:
**08.02.2023 Bulletin 2023/06**

(73) Proprietor: **Universiteit Gent**
**9000 Gent (BE)**

(72) Inventors:
- **VERHELST, Xavier**
  **9860 Landskouter (BE)**
- **VAN VLIERBERGHE, Hans**
  **9750 Kruisem (BE)**

(56) References cited:
- **DECAENS T ET AL: "Factors associated with tumor recurrence after liver transplantation for hepatocellular carcinoma: prospective cohort on 371 patients", JOURNAL OF HEPATOLOGY, vol. 66, no. 1, 2017, XP085011823, ISSN: 0168-8278, DOI: 10.1016/S0168-8278(17)30694-3**
- **VERHELST XAVIER ET AL: "Glycome Patterns of Perfusate in Livers Before Transplantation Associate With Primary Nonfunction", GASTROENTEROLOGY : OFFICIAL PUBLICATION OF THE AMERICAN GASTROENTEROLOGICAL ASSOCIATION, WILLIAMS & WILKINS, US, vol. 154, no. 5, 6 January 2018 (2018-01-06), pages 1361 - 1368, XP085372513, ISSN: 0016-5085, DOI: 10.1053/J.GASTRO.2017.12.027**
- **LIU X-E ET AL: "N-Glycomic Changes in Hepatocellular Carcinoma Patients with Liver Cirrhosis Induced by Hepatitis B Virus", HEPATOLOGY, JOHN WILEY & SONS, INC, US, vol. 46, no. 5, 1 January 2007 (2007-01-01), pages 1426 - 1435, XP002494797, ISSN: 0270-9139, DOI: 10.1002/HEP.21855**
- **XAVIER VERHELST ET AL: "A Glycomics-Based Test Predicts the Development of Hepatocellular Carcinoma in Cirrhosis", CLINICAL CANCER RESEARCH, vol. 23, no. 11, 16 December 2016 (2016-12-16), US, pages 2750 - 2758, XP055691101, ISSN: 1078-0432, DOI: 10.1158/1078-0432.CCR-16-1500**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical field of invention

The present invention relates to the technical field of predicting recurrence of hepatocellular carcinoma (HCC) after liver transplantation. The present invention discloses a process to predict the recurrence of HCC after liver transplantation via determining the amount of at least four specific N-glycans in a serum sample.

### Background art

Liver transplantation is the ultimate treatment for end stage liver disease and selected patients with hepatocellular carcinoma. Only patients with HCC limited to the liver (without extrahepatic disease) and responding to strict criteria regarding the number and size of the HCC lesions (e.g. Milan-Criteria, Up-to-seven Criteria,...) can be considered for liver transplantation in order to limit the risk of disease recurrence after transplantation. However, although these criteria show a strong correlation with disease recurrence, sensitivity and specificity should be increased, as recurrence still occurs despite stringent application of these criteria. This could be explained by underestimation of the number of lesions using current imaging modalities and because tumour biology is not taken into account in these models. Decaens et al. 2017 (J. Hepatology 66, abstract THU-464) discloses alpha-foetoprotein as a (blood-derived) biomarker for predicting tumor recurrence after liver transplantation in HCC patients. The recent Duvoux-model (Duvoux et al. 2012) takes into account alpha-foetoprotein, a diagnostic marker for HCC, to increase the predictive power of HCC recurrence. On the other hand, some patients are denied liver transplantation based on these models, although their risk of recurrence of HCC after liver transplantation could be low.

There is thus a clear need for prognostic biomarkers that assess the risk of HCC recurrence after liver transplantation, beyond the current models.

Miyahara et al. (2014 & 2015) and Kamiyama et al. (2013) disclose that specific serum glycans in patients with HCC present before treatment is initiated, are independently associated with HCC recurrence and overall survival. However, it has not been studied and is unknown whether and which set of serum glycans can be efficiently used to predict with high certainty the recurrence of HCC after liver transplantation.

### Brief description of figures

Figure 1. Cumulative risk of HCC recurrence after LT according to HCC Recurrence Score LR. Log Rank test : p = 0.017.

Figure 2. Cumulative risk of HCC recurrence after LT according to HCC Recurrence Score CR. Log Rank test : p = 0.017.

### Description of invention

The present invention relates in first instance to a process to predict recurrence of hepatocellular carcinoma after liver transplantation comprising:

- providing a serum or plasma sample obtained from a hepatocellular carcinoma patient before said patient receives a liver transplant,
- determining the amount of the N-glycans: agalacto, core-alpha-1,6-fucosylated biantennary glycan (NGA2F), an agalacto, core-alpha-1,6-fucosylated bisecting biantennary (NGA2FB), an unfucosylated triantennary glycan (NA3), and a branching alpha-1,3-fucosylated and core-alpha-1,6-fucosylated triantennary glycan (NA3Fbc) within said serum or plasma sample,

wherein decreased amounts of NGA2F and NGA2FB, and, increased amounts of NA3 and NA3Fbc -compared to the amounts of NGA2F, NGA2FB, NA3 and NA3Fbc within a control serum sample- are predictive for recurrence of hepatocellular carcinoma after liver transplantation.

The terms 'to predict recurrence of hepatocellular carcinoma after liver transplantation' relates to forecasting in a reliable manner whether a recipient -who has/had HCC before transplantation- will develop HCC after transplantation.

The terms 'providing a serum or plasma sample obtained from a HCC patient' relate to well-known practices to obtain a blood sample -and subsequently a serum or plasma sample- from a human being. The present invention thus relates to an 'in vitro' diagnostic method applied on a serum or plasma sample after said serum/plasma sample has been

removed from a human body.

The term agalacto, core-alpha-1,6-fucosylated biantennary glycan (NGA2F) refers to the following oligosaccharide-type structure:

(XI)

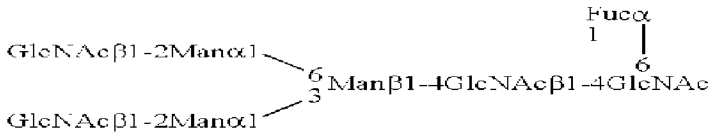

The term agalacto, core-alpha-1,6-fucosylated bisecting biantennary glycan (NGA2FB) relates to the following oligosaccharide-type structure :

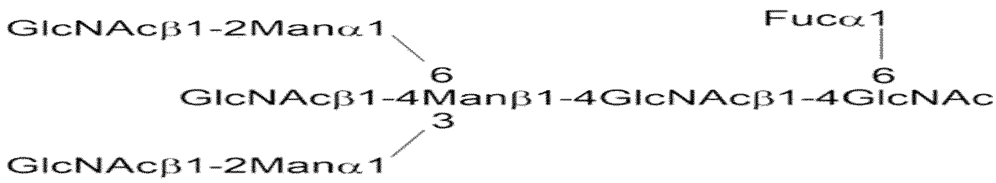

The term unfucosylated triantennary glycan (NA3) relates to the following oligosaccharide-type structure :

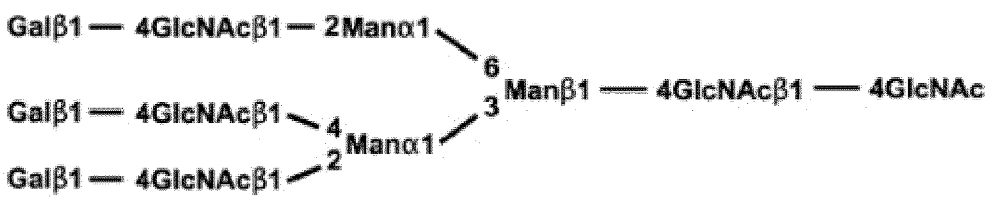

The term branching alpha-1,3 fucosylated and core-alpha-1,6-fucosylated triantennary glycan (NA3Fbc) relates to the following oligosaccharide-type structure :

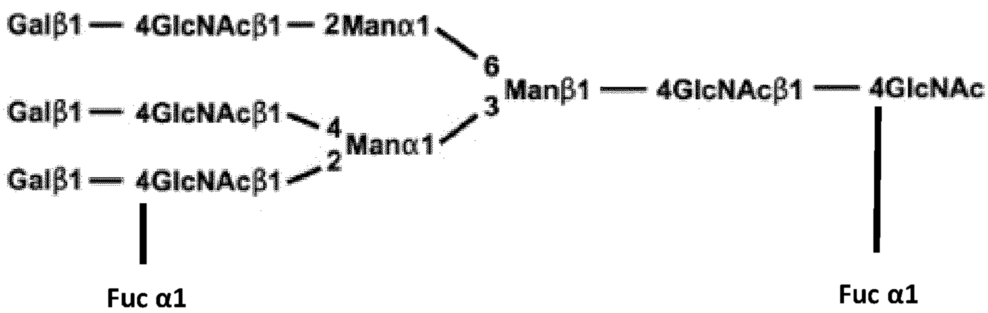

The present invention further relates to a process as describe above wherein , in addition, the amount of core-alpha-1,6-fucosylated triantennary glycan (NA3Fc) is determined within said serum/plasma sample and wherein an increased amounts of NA3Fc -compared to the amounts of NA3Fc within a control serum/plasma sample- is predictive for recurrence of hepatocellular carcinoma after liver transplantation.

The term core-alpha-1,6-fucosylated triantennary glycan (NA3Fc) relates to the following oligosaccharide-type structure :

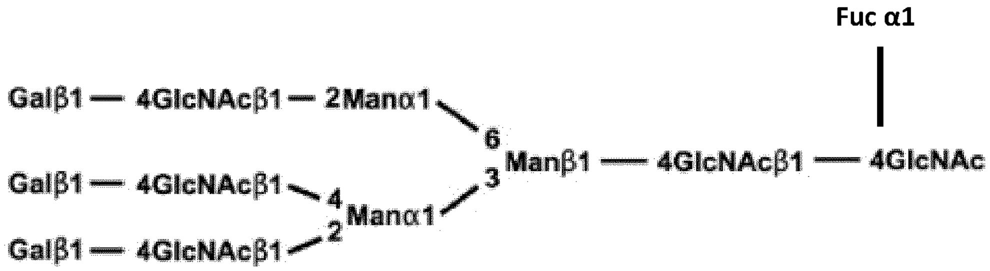

The terms "determining the amount of the N-glycans: agalacto, core-alpha-1,6-fucosylated biantennary (NGA2F), agalacto, core-alpha-1,6-fucosylated bisecting biantennary (NGA2FB), unfucosylated triantennary glycan (NA3), branching alpha-1,3-fucosylated and core alpha-1,6-fucosylated triantennary glycan (NA3Fbc) and core-alpha-1,6-fucosylated triantennary glycan (NA3Fc) within said serum sample" ' relates to any method known to (relatively) quantify the presence of NGA2F, NGA2FB, NA3, NA3Fbc and/or NA3Fc molecules within said serum sample. Specifically, the latter terms refer to 'determining the amount of NGA2F, NGA2FB, NA3, NA3Fbc and/or NA3Fc via capillary electrophoresis' and more specifically the latter terms refer to 'determining the amount of NGA2F, NGA2FB, NA3, NA3Fbc and/or NA3Fc via DNA sequencer-assisted fluorophore-assisted capillary electrophoresis' as is described by Callewaert et al. (2004 Nat Med 10: 429). Other techniques can be used for quantification/profiling of glycans including MALDI-TOF Mass spectometry, high-performance anion-exchange chromatography with pulsed amperometric detection, High Performance Liquid chromatography and Lectin Array.

The preparation and labeling of the glycans in serum sample for sequencing can be performed using the on-membrane deglycosylation method described by Laroy et al. (Nat. Protoc. 1, 397-405 (2006)). An in-solution deglycosylation method has been developed (Laroy et al. 2006) for serum glycan profiling in a clinical context (sample preparation time less than 2 hours).

The present invention thus specifically relates to a process as described above wherein said determining the amount of said glycans within said serum/plasma sample is undertaken by capillary electrophoresis. Moreover, the present invention relates to a process as described above wherein said capillary electrophoresis is DNA sequencer-assisted fluorophore-assisted capillary electrophoresis.

The present invention further relates to a process as described above wherein said control serum/plasma sample is a serum/plasma sample obtained from a hepatocellular carcinoma patient before said patient receives a liver transplant and wherein said patient experiences no recurrence of hepatocellular carcinoma after liver transplantation.

The present invention thus relates to the usage of the N-glycans NGA2F, NGA2FB, NA3, NA3Fbc and/or NA3Fc to predict recurrence of hepatocellular carcinoma after liver transplantation according to a process as described above. Based on changes in relative abundance of glycans in patients who show recurrent HCC after liver transplantation, the following scoring systems can for example be used: a "HCC Recurrence Score LR" which calculates a risk score using a logistic regression model, or, a "HCC Recurrence Score CR" which calculates a risk score using a cox regression model.

Based on the latter Recurrence Scores a ROC curve analysis can be undertaken and -based on for example the Youden-index- a cut-off with the best matching sensitivity and specificity can be calculated.

The present invention will now be further illustrated by the following non-limiting examples.

Examples

In a prospective monocentric study in an expert liver transplant unit, consecutive serum samples were collected the day before liver transplantation (LT) in 255 patients between 2 July 2011 and 24 September 2018. In this cohort, 76 patients were diagnosed having HCC. Patients were followed after LT for at least one year. Serum glycomic analysis was performed as described by Callewaert et al. and Laroy et al. using capillary electrophoresis. This yields an electropherogram with 13 peaks covering the entire spectrum from agalactosylated biantennary glycans to complex tetra-antennary glycans. The relative abundance of these glycans is normalized over the total amount of measured glycans in the serum sample.

In this cohort, 8 patients developed HCC recurrence during follow up. The pre-transplant glycomic profile of these patients was significantly different of patients with HCC who did not develop HCC recurrence after liver transplantation.

Using both logistic and cox regression, a specific glycomic fingerprint was observed as described before, and summarized in table 1 and 2.

Table 1. Univariate Logistic Regression analysis of changes in abundance of serum glycans in patients with recurrent HCC after liver transplantation compared to HCC patients without recurrence after liver transplantation.

| | Regressie coefficient | OR | p value | 95% CI | 95% CI |
|---|---|---|---|---|---|
| **NGA2F** | -0.036 | 0.965 | 0.011 | 0.965 | 0.992 |
| **NGA2FB** | -0.17 | 0.863 | 0.009 | 0.772 | 0.964 |
| **NG1A2F** | -0.04 | 0.960 | 0.073 | 0.918 | 1.004 |
| **NG1A2Fisomer** | 0.013 | 1.013 | 0.655 | 0.957 | 1.073 |
| **NA2** | 0.004 | 1.004 | 0.377 | 0.996 | 1.012 |
| **NA2F** | 0.002 | 1.003 | 0.803 | 0.983 | 1.023 |
| **NA2FB** | -0.024 | 0.976 | 0.071 | 0.951 | 1.002 |
| **NA3** | 0.033 | 1.034 | 0.010 | 1.008 | 1.060 |
| **NA3Fb** | 0.027 | 1.028 | 0.259 | 0.980 | 1.079 |
| **NA3Fc** | 0.085 | 1.202 | 0.003 | 1.018 | 1.419 |
| **NA3Fbc** | 0.071 | 1.075 | 0.031 | 1.006 | 1.147 |
| **NA4** | 0.050 | 1.054 | 0.612 | 0.860 | 1.293 |
| **NA4Fb** | -0.072 | 0.930 | 0.675 | 0.663 | 1.304 |

Table 2. Univariate Cox Regression analysis of changes in abundance of serum glycans in patients with recurrent HCC after liver transplantation compared to HCC patients without recurrence after liver transplantation.

| | Regressie coefficient | HR | p value | 95% CI | 95% CI |
|---|---|---|---|---|---|
| **NGA2F** | -0.029 | 0.972 | 0.018 | 0.949 | 0.995 |
| **NGA2FB** | -0.110 | 0.896 | 0.013 | 0.822 | 0.977 |
| **NG1A2F** | -0.033 | 0.967 | 0.106 | 0.929 | 1.007 |
| **NG1A2Fisomer** | 0.011 | 1.011 | 0.684 | 0.959 | 1.066 |
| **NA2** | 0.002 | 1.002 | 0.538 | 0.995 | 1.010 |
| **NA2F** | 0.001 | 1.001 | 0.873 | 0.984 | 1.019 |
| **NA2FB** | -0.018 | 0.982 | 0.107 | 0.960 | 1.004 |
| **NA3** | 0.020 | 1.020 | 0.001 | 1.008 | 1.032 |
| **NA3Fb** | 0.022 | 1.022 | 0.303 | 0.980 | 1.066 |
| **NA3Fc** | 0.004 | 0.996 | 0.732 | 0.974 | 1.018 |
| **NA3Fbc** | 0.034 | 1.035 | 0.009 | 1.009 | 10.62 |
| **NA4** | 0.036 | 1.036 | 0.703 | 0.863 | 1.245 |
| **NA4Fb** | -0.028 | 0.972 | 0.847 | 0.732 | 1.291 |

Calculation of the HCC Recurrence Score Log Reg and the HCC Recurrence Score Cox Reg:

Based on this information first an HCC recurrence Score based on logistic regression was calculated as:

HCC Recurrence Score Log Reg = (NGA2F*( -0,036)) + (NGA2FB*(-0,147)) + (NA3*0,033) + (NA3Fc *0,182) + (NA3Fbc*0,071)

A similar score was developed using Cox regression analysis:

HCC Recurrence Score Cox Reg= (NGA2F*(-0,029)) + (NGA2FB*(-0,110)) + (NA3*0,020) + (NA3Fc *0,031) + (NA3Fbc*0,034)

ROC curve analysis showed an AUC of 0.866 (p=0.001; 95%CI 0.750-0.982) for the HCC Recurrence Score Log Reg and an AUC of 0.855 (p=0.001; 95%CI 0.731-0.979) for the HCC Recurrence Score Cox Reg for the development of recurrent HCC after liver transplantation.

Based on the Youden index, a cut-off of -3.2788 was defined for the HCC Recurrence Score Log Reg showing a sensitivity of 87,5% and a specificity of 73,5%.

For the HCC Recurrence Score Cox Reg, a cut-off of -4.24 was defined using a similar approach. Sensitivity was 87.5% and specificity 67.6%.

Using this cut-off, the Kaplan Meier curve showed a significant difference for the HCC Recurrence Score Log Reg score (Log Rank test: p=0.001) between patients with and without HCC recurrence. The cumulative incidence of HCC recurrence was 2% in patients below this cutoff and 28% in patients with a pre-transplant value above this cutoff.

When the HCC Recurrence Score Cox Reg was applied, the cumulative incidence of HCC was 2.1% after liver transplantation if the value was below -4.24, and 24.1% if the value was equal to or higher than this cut-off (Log Rank t test: p=0.011).

In a next step, we calculated the association of these scoring systems with HCC recurrence without using NA3Fc. This shows the independent and strong association of this glycomic signature with HCC recurrence after liver transplantation without the use of NA3Fc.

$$\text{HCC Recurrence Score LR} = (NGA2F*(-0.036))+(NGA2FB*(-0.17))+(NA3*0.033)+(NA3Fbc*0.071)$$

ROC curve analysis for the "HCC recurrence score LR" showed an area under the curve (AUC) of 0.837 (p=0.002; 95% Cl 0.711-0.963). The Youden-index was used to calculate a cut-off with the best matching sensitivity and specificity. This was defined at -5.80 associated with a sensitivity of 87.5% and specificity of 68.1% for HCC recurrence after LT.

Dividing the cohort according to this cut-off shows a clear differentiation according to HCC recurrence risk (figure 1).

$$\text{HCC Recurrence Score CR} = (NGA2F*(-0.029))+(NGA2FB*(-0.110))+(NA3*0.020)+(NA3Fbc*0.034)$$

ROC curve analysis for the "HCC recurrence score CR" showed an area under the curve (AUC) of 0.837 (p=0.002; 95% Cl 0.709-0.965). The Youden-index was used to calculate a cut-off with the best matching sensitivity and specificity. This was defined at -4.54 associated with a sensitivity of 87.5% and specificity of 68.1% for HCC recurrence after LT.

Dividing the cohort according to this cut-off shows a clear differentiation according to HCC recurrence risk (figure 2).

References

- Duvoux C, Roudot-Thoraval F, Decaens T, Pessione F, Badran H, Piardi T, Francoz C, Compagnon P, Vanlemmens C, Dumortier J, Dharancy S, Gugenheim J, Bernard PH, Adam R, Radenne S, Muscari F, Conti F, Hardwigsen J, Pageaux GP, Chazouillères O, Salame E, Hilleret MN, Lebray P, Abergel A, Debette-Gratien M, Kluger MD, Mallat A, Azoulay D, Cherqui D; Liver Transplantation French Study Group. Liver transplantation for hepatocellular carcinoma: a model including $\alpha$-fetoprotein improves the performance of Milan criteria. Gastroenterology. 2012 Oct;143(4): 986-94.

- Miyahara et al. Alteration of N-glycan profiles in patients with chronic hepatitis and hepatocellular carcinoma. Hepatology Research 2015: 986.

- Miyahara et al. Serum glycan as a prognostic marker in patients with advanced hepatocellular carcinoma treated with sorafenib. Hepatology 2014: 355.

- Kamayama et al. Identification of novel serum biomarkers of hepatocellular carcinoma using glycomic analysis. Hepatology 2013: 2314.

- Callewaert, N. et al. Noninvasive diagnosis of liver cirrhosis using DNA sequencer-based total serum protein glycomics. Nat. Med. 10, 429-34 (2004).

- Laroy, W., Contreras, R. & Callewaert, N. Glycome mapping on DNA sequencing equipment. Nat. Protoc. 1, 397-405 (2006).


## Claims

**1.** A process to predict recurrence of hepatocellular carcinoma after liver transplantation comprising:

- providing a serum or a plasma sample obtained from a hepatocellular carcinoma patient before said patient receives a liver transplant,
- determining the amount of the N-glycans: agalacto, core-alpha-1,6-fucosylated biantennary (NGA2F), agalacto, core-alpha-1,6-fucosylated bisecting biantennary (NGA2FB), triantennary glycan (NA3), and branching alpha-1,3-fucosylated and core-alpha-1,6-fucosylated triantennary glycan (NA3Fbc) within said serum or plasma sample,

wherein decreased amounts of NGA2F and NGA2FB, and, increased amounts of NA3 and NA3Fbc -compared to the amounts of NGA2F, NGA2FB, NA3 and NA3Fbc within a control serum or plasma sample- are predictive for recurrence of hepatocellular carcinoma after liver transplantation.

**2.** A process according to claim 1 wherein , in addition, the amount of core-alpha-1,6-fucosylated triantennary glycan (NA3Fc) is determined within said serum or plasma sample and wherein an increased amounts of NA3Fc -compared to the amounts of NA3Fc within a control serum or plasma sample- is predictive for recurrence of hepatocellular carcinoma after liver transplantation.

**3.** A process according to claims 1 and 2 wherein said determining the amount of said glycans within said serum or plasma sample is undertaken by capillary electrophoresis.

**4.** A process according to claim 3 wherein said capillary electrophoresis is DNA sequencer-assisted fluorophore-assisted capillary electrophoresis.

**5.** A process according to any of claims 1-4 wherein said control serum or plasma sample is a serum or a plasma sample obtained from a hepatocellular carcinoma patient before said patient receives a liver transplant and wherein said patient experiences no recurrence of hepatocellular carcinoma after liver transplantation.

## Patentansprüche

**1.** Verfahren zur Vorhersage eines Leberzellkarzinom-Rezidivs nach Lebertransplantation, umfassend:

- Bereitstellen einer Serum- oder Plasmaprobe, die einem Patienten mit Leberzellkarzinom entnommen wird, bevor dieser ein Lebertransplantat erhält,
- Bestimmen der Menge der N-Glycane NGA2F (agalacto, kern-alpha-1,6-fucosyliert, biantennär), NGA2FB (agalacto, kern-alpha-1,6-fucosyliert, *bisecting,* biantennär), NA3 (triantennäres Glycan) und NA3Fbc (verzweigend alpha-1,3-fucosyliertes und kern-alpha-1,6-fucosyliertes triantennäres Glycan) in der Serum- bzw. Plasmaprobe,

wobei verminderte Mengen an NGA2F und NGA2FB und erhöhte Mengen an NA3 und NA3Fbc - verglichen mit den Mengen an NGA2F, NGA2FB, NA3 und NA3Fbc in einer Kontrollserum- bzw. -plasmaprobe - ein Leberzellkarzinom-Rezidiv nach Lebertransplantation vorhersagen.

**2.** Verfahren nach Anspruch 1, wobei zusätzlich die Menge an NA3Fc (kern-alpha-1,6-fucosyliertes triantennäres Glycan) in der Serum- oder Plasmaprobe bestimmt wird und wobei eine erhöhte Menge an NA3Fc - verglichen mit den

Mengen an NA3Fc in einer Kontrollserum- bzw. -plasmaprobe - ein Leberzellkarzinom-Rezidiv nach Lebertransplantation vorhersagt.

**3.** Verfahren nach Anspruch 1 und 2, wobei das Bestimmen der Menge der Glykane in der Serum- oder Plasmaprobe mit Kapillarelektrophorese erfolgt.

**4.** Verfahren nach Anspruch 3, wobei es sich bei der Kapillarelektrophorese um DNA-Sequenzierautomatgestützte fluorophorgestützte Kapillarelektrophorese handelt.

**5.** Verfahren nach einem der Ansprüche 1-4, wobei es sich bei der Kontrollserum- oder -plasmaprobe um eine Serum- oder eine Plasmaprobe handelt, die einem Patienten mit Leberzellkarzinom entnommen wird, bevor dieser ein Lebertransplantat erhält, und wobei bei dem Patienten nach Lebertransplantation kein Leberzellkarzinom-Rezidiv auftritt.

**Revendications**

**1.** Procédé pour prédire la récurrence d'un carcinome hépatocellulaire après transplantation hépatique comprenant :

- la fourniture d'un échantillon de sérum ou de plasma obtenu d'un patient atteint d'un carcinome hépatocellulaire avant que ledit patient ne reçoive une greffe du foie,
- la détermination de la quantité de N-glycanes : agalacto, biantennaire à noyau alpha-1,6-fucosylé (NGA2F), agalacto, biantennaire bissecteur à noyau alpha-1,6-fucosylé (NGA2FB), glycane triantennaire (NA3) et glycane triantennaire alpha-1,3-fucosylé ramifiant et à noyau alpha-1,6-fucosylé (NA3Fbc) dans ledit échantillon de sérum ou de plasma,

dans lequel des quantités réduites de NGA2F et NGA2FB, et des quantités accrues de NA3 et NA3Fbc - comparées aux quantités de NGA2F, NGA2FB, NA3 et NA3Fbc dans un échantillon de sérum ou de plasma témoin - sont prédictives de la récurrence d'un carcinome hépatocellulaire après transplantation hépatique.

**2.** Procédé selon la revendication 1, dans lequel, en outre, la quantité de glycane triantennaire à noyau alpha-1,6-fucosylé (NA3Fc) est déterminée dans ledit échantillon de sérum ou de plasma et dans lequel une quantité accrue de NA3Fc - comparée aux quantités de NA3Fc dans un échantillon de sérum ou de plasma témoin - est prédictive de la récurrence d'un carcinome hépatocellulaire après transplantation hépatique.

**3.** Procédé selon les revendications 1 et 2, dans lequel ladite détermination de la quantité desdits glycanes dans ledit échantillon de sérum ou de plasma est entreprise par électrophorèse capillaire.

**4.** Procédé selon la revendication 3, dans lequel ladite électrophorèse capillaire est une électrophorèse capillaire assistée par fluorophore assistée par séquenceur d'ADN.

**5.** Procédé selon l'une quelconque des revendications 1-4 dans lequel ledit échantillon de sérum ou de plasma témoin est un échantillon de sérum ou de plasma obtenu d'un patient atteint d'un carcinome hépatocellulaire avant que ledit patient ne reçoive une greffe du foie et dans lequel ledit patient ne présente aucune récurrence d'un carcinome hépatocellulaire après une transplantation hépatique.

Figure 1

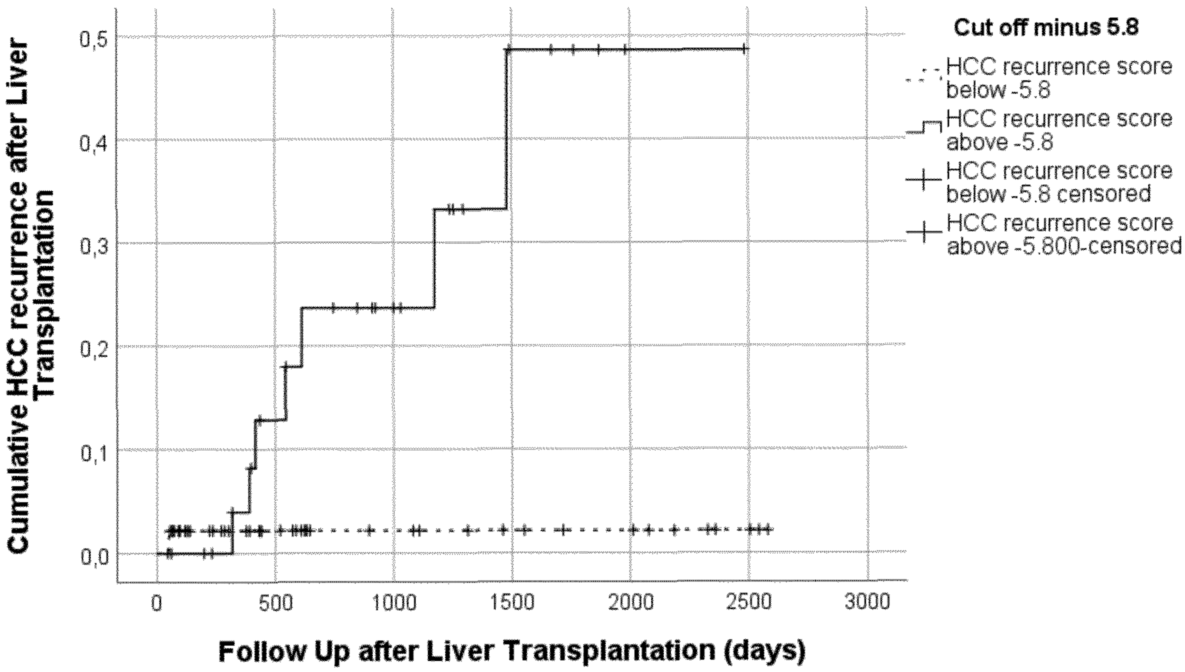
Cumulative HCC recurrence after Liver Transplantation
0,5
0,4
0,3
0,2
0,1
0,0
0
500
1000
1500
2000
2500
3000
Follow Up after Liver Transplantation (days)
Cut off minus 5.8
HCC recurrence score below -5.8
HCC recurrence score above -5.8
HCC recurrence score below -5.8 censored
HCC recurrence score above -5.800-censored

Figure 2

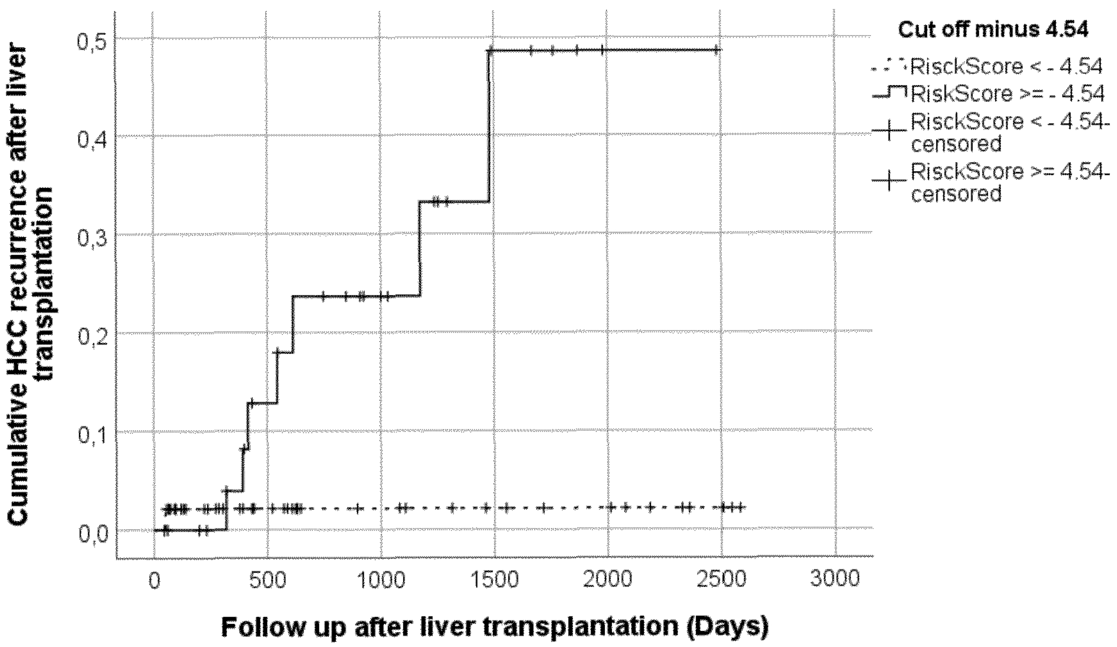
Cumulative HCC recurrence after liver transplantation
0,5
0,4
0,3
0,2
0,1
0,0
0
500
1000
1500
2000
2500
3000
Follow up after liver transplantation (Days)
Cut off minus 4.54
RisckScore < - 4.54
RiskScore >= - 4.54
RisckScore < - 4.54-censored
RisckScore >= 4.54-censored

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DECAENS et al.** *J. Hepatology*, 2017, vol. 66
- **CALLEWAERT et al.** *Nat Med*, 2004, vol. 10, 429
- **LAROY et al.** *Nat. Protoc.*, 2006, vol. 1, 397-405
- **DUVOUX C** ; **ROUDOT-THORAVAL F** ; **DECAENS T** ; **PESSIONE F** ; **BADRAN H** ; **PIARDI T** ; **FRANCOZ C** ; **COMPAGNON P** ; **VANLEMMENS C** ; **DUMORTIER J**. Liver transplantation for hepatocellular carcinoma: a model including $\alpha$-fetoprotein improves the performance of Milan criteria.. *Gastroenterology*, October 2012, vol. 143 (4), 986-94
- **MIYAHARA et al.** Alteration of N-glycan profiles in patients with chronic hepatitis and hepatocellular carcinoma.. *Hepatology Research*, 2015, 986
- **MIYAHARA et al.** Serum glycan as a prognostic marker in patients with advanced hepatocellular carcinoma treated with sorafenib.. *Hepatology*, 2014, 355
- **KAMAYAMA et al.** Identification of novel serum biomarkers of hepatocellular carcinoma using glycomic analysis. *Hepatology*, 2013, 2314
- **CALLEWAERT, N. et al.** Noninvasive diagnosis of liver cirrhosis using DNA sequencer-based total serum protein glycomics. *Nat. Med.*, 2004, vol. 10, 429-34
- **LAROY, W.** ; **CONTRERAS, R.** ; **CALLEWAERT, N.** Glycome mapping on DNA sequencing equipment. *Nat. Protoc.*, 2006, vol. 1, 397-405